# FASCICULE DE BREVET EUROPEEN

(11) **EP 0 686 194 B1**
(45) Date de publication et mention de la délivrance du brevet: **09.07.2003**
(21) Numéro de dépôt: 94909145.8
(22) Date de dépôt: 25.02.1994
(51) Int. Cl.: C12N 15/12, C07K 14/475, A61K 38/18

(54) **FACTEURS DE CROISSANCE DE LA FAMILLE DE L'HARP, PROCEDE D'OBTENTION ET APPLICATIONS**
WACHTUMSFAKTOR AUS DER HARP-REIHE, VERFAHREN ZU IHRER HERSTELLUNG UND VERWENDUNGEN DAVON
HARP FAMILY GROWTH FACTORS, PREPARATION METHODS THEREFOR AND USES THEREOF

(30) Priorité: 26.02.1993 FR 9302270
(43) Date de publication de la demande: 13.12.1995
(73) Titulaire: Valbiofrance, 75006 Paris (FR)
(72) Inventeur: BARRITAULT, Denis, F-75001 Paris (FR); COURTY, José, F-94440 Villecresnes (FR); LAAROUBI, Khalid, Sidi Kacem (MA)
(74) Mandataire: Michelet, Alain
(86) Numéro de dépôt international: FR9400219
(87) Numéro de publication internationale: WO94019462

(56) Documents cités:
- EP-A- 0 474 979
- SCIENCE. vol. 250 , 21 Décembre 1990 , LANCASTER, PA US pages 1690 - 1693 YUE-SHENG LI ET AL. 'Cloning and expression of a developmentally regulated protein that induces mitogenic and neurite outgrowth activity' cité dans la demande

## Description

La présente invention a pour objet un facteur de croissance de la famille de l'HARP.

Elle est également relative à des procédés d'obtention de ce facteur en mettant en oeuvre des techniques d'ingénierie génétique.

Elle concerne aussi les applications d'un tel facteur en thérapeutique.

Des facteurs de croissance appelés HBBM ont été mis en évidence lors de la purification d'autres facteurs de croissance , du groupe des FGF_{S} (Fibroblast Growth Factor).

Les HBBM ont fait notamment l'objet d'une demande de brevet européen 89 101 187 ( N° de publication EP-326.075) dans laquelle on décrit leur isolement à partir d'un extrait de cerveau , par un procédé de purification incluant une extraction à pH acide et des étapes de chromatographie. Ce procédé a permis d'isoler trois peptides de 18 kD, 16 kD et 15 kD ayant la même extrémité terminale avec la séquence NH₂-GLY-LYS-LYS-GLU-LYS-PRO-GLU-LYS-LYS-VAL-LYS-LYS-SER-ASP-CYS-GLY-GLU-TRP-GLN-TRP-SER-VAL-CYS-VAL-PRO.

La demande EP-326.075 décrit une activité mitogène des HBBM sur des cellules endothéliales à des doses de l'ordre de 20 à 50 ng/ml, pour l'induction d'un effet de stimulation de 50% de la stimulation maximale de la prolifération, et un effet minimum à partir de 3 ng/ml . Dans ce dosage, l'effet mitogène des HBBM est comparable à celui de la forme acide du FGF (aFGF) . Cette demande mentionne aussi pour les trois formes HBBM une activité de promotion de l'angiogenèse et des activités d'entretien de l'intégrité et de cicatrisation tissulaire pour les tissus cutanés, osseux et nerveux notamment , sans que des résultats expérimentaux soient rapportés.

La demande de brevet européen publiée sous le n° EP 474,979 décrit un polypeptide HBNF codé par un ADNc obtenu à partir de cerveau de rat. Le polypeptide HBNF mature comprend 136 acides aminés de longueur et débuté par la séquence d'acides aminés N-terminale « NH₂-GLY-LYS-LYS-GLU-LYS-PRO-GLU-LYS-LYS-VAL ».

Dans un article ultérieur ( Growth Factors, 4: 97-107, 1991), les inventeurs reviennent sur les résultats décrits dans le brevet EP-326.075 et démontrent que leurs nouvelles préparations d'HBBM également obtenues à partir de cerveau de boeuf, sont en réalité dépourvues d'activité mitogène, notamment pour des cellules endothéliales et fibroblastiques, même à des doses de 1 à 10 µg/ml. Seule est confirmée une activité neurotrophique donnant une croissance neuritique sur des neurones d'embryons de rat à des doses comprises entre 80 et 640 ng/ml. Les auteurs de cet article décident donc de changer le nom de ce facteur pour le désigner par le nouveau sigle HBNF pour "Heparin Binding Neurotrophic Factor". La raison de ces différences entre les premiers travaux décrits dans.la demande de brevet et ceux décrits ultérieurement n'est pas clairement comprise mais l'hypothèse avancée est. celle d'une contamination par le facteur de croissance bFGF lors des premières préparations. En effet, le facteur bFGF est déjà bien connu pour ses activités angiogénique et mitogène et s'isole par une procédure similaire à partir du cerveau.

Afin de confirmer leurs résultats sur l'absence d'activités mitogène et angiogénique du HBNF, les mêmes auteurs purifient le HBNF à partir du produit d'expression du gène de cette protéine dans des bactéries ( Kretschmer et al., Growth Factors , 5: 99-114, 1991). La protéine obtenue contient 168 acides aminés et est purifiée après maturation sous la forme d'une protéine de 136 acides aminés, dont la séquence est identique à celle décrite précédemment, commençant donc du côté N-terminal par les acides aminés NH₂-GLY-LYS-LYS-GLU-LYS-PRO-GLU-LYS-LYS-VAL. Cette protéine est dépourvue d'activités mitogène et angiogénique . Seule l'activité neurotrophique, comparable à celle de la protéine HBNF purifiée à partir du cerveau, est confirmée .

Par une approche comparable, une autre équipe de chercheurs démontre que la même protéine obtenue par expression du même gène , appelé par ces auteurs HB-GAM et inséré sur un vecteur baculovirus dans des cellules d'insectes, est également dépourvue d'activité mitogène (Raulo et al., J. Biol. Chem., 267, 1-9, 1992). La protéine ainsi purifiée a une séquence N-terminale GLY-LYS-LYS-GLU-LYS identique à celle que ces auteurs ont obtenue à partir d'une extraction de cerveau de rat. Cette protéine recombinante a une activité neurotrophique comparable à la protéine native . Les auteurs concluent que seule l'activité neurotrophique de HB-GAM existe. Elle est optimale à 200 ng/ml dans le test utilisé sur des cellules neuronales cultivées sur un support recouvert de la protéine HB-GAM.

Des résultats analogues, montrant l'absence d'activité mitogène de cette protéine, extraite de cerveaux de boeuf, sont également rapportés par d'autres chercheurs travaillant indépendamment , voir Molecular Biology of the Cell, 3: 85-93, 1992 et J. Biol. Chem., 265: 18749-18752, 1990 . L'activité neurotrophique est également décrite . La séquence peptidique est identique à HB-GAM ou HBNF avec du côté N-terminal les acides aminés GLY-LYS-LYS-GLU-LYS.

Parallèlement , à partir d'utérus de bovin, a été extraite la même protéine ( séquence N-terminale GLY-LYS-LYS-GLU-LYS) , dénommée HBGF-8 par les auteurs , puis Pléiotrophine (PTN), ayant une activité mitogène sur fibroblastes (Biochem. Biophys. Res. Commun., 165: 1096-1103, 1989). Une année plus tard, les mêmes auteurs ( Li et al, Science, 250: 1690, 1990) publient les résultats d'une expérimentation de transfection de cellules eucaryotes COS-7 et une expression transitoire du gène PTN. Ces auteurs montrent que le lysat cellulaire des cellules COS-7 transfectées par le gène PTN est capable d'induire la multiplication cellulaire . Cependant la caractérisation moléculaire de la protéine PTN n'a pas été réalisée .

D'autres scientifiques , encore , ont montré que des cellules tumorales d'origine mammaire sécrètent un facteur de croissance capable d'être retenu sur une chromatographie d'héparine-Sépharose. Ce facteur a été purifié et sa séquence peptidique N-terminale est identique à celle de l'HARP à l'exception toutefois du premier acide aminé qui n'est pas déterminé (WELLSTEIN et al. J. Biol. Chem. 267: 2582, 1992).

Il ressort donc de l'état de la technique analysé ci-dessus qu'il existe une grande incertitude quant à la structure de la ou des molécules responsables des activités mitogènes , angiogéniques et neurotrophiques décrites par les différents auteurs.

L'homme du métier pouvait néanmoins supposer , au vu de ces résultats , que la ou les molécules isolées par les diverses équipes possédai(en)t une extrémité N-terminale commune ayant la séquence suivante :

La demanderesse s'est donc attachée à trouver des molécules de structures bien déterminées, possédant les activités précitées à des valeurs permettant l'utilisation thérapeutique.

Elle s'est également attachée à mettre au point un procédé d'obtention de ces molécules sous une forme substantiellement libre de contaminant.

Elle a trouvé de manière surprenante que ces molécules présentaient des séquences en partie similaires à celles déjà décrites, mais allongées par des acides aminés à son extrémité N-terminale , et des activités biologiques supérieures à celles décrites dans l'état de la technique.

Elle a d'autre part montré que l'on pouvait obtenir ces molécules dans des quantités suffisantes et sous une forme quasiment pure , par insertion et expression d'un ADN complémentaire de cette molécule dans des cellules eucaryotes ou dans des bactéries.

La présente invention a donc pour objet un peptide facteur de croissance ayant une masse de 18 KD
ledit peptide étant caractérisé en ce que sa séquence est la séquence SEQ is N° 2 suivante :

Le peptide facteur de croissance de séquence SEQ is N°2 est d'autant plus surprenant qu'il était difficile pour l'homme du métier , ayant connaissance de l'état de la technique analysé ci-dessus, de prédire qu'une adjonction d'acides aminés à la séquence N-terminale du peptide connu , améliorerait considérablement son activité biologique.

En effet, les prédictions quant à l'influence de l'adjonction, de l'élimination ou de la modification d'un acide aminé dans une structure donnée sont impossibles dans l'état actuel des connaissances de la structure des protéines, même à l'aide des méthodes de modélisation les plus avancées.

La présente invention a encore pour objet l'obtention du peptide tel que défini précédemment par la technique générale d'ingénierie génétique , selon laquelle des vecteurs portant un ADN complémentaire codant ledit peptide de séquence SEQ is Nº 2, sont exprimés et le peptide est ensuite extrait .

Le procédé d'obtention du peptide tel que défini précédemment comprend une étape de sécrétion dudit peptide dans les milieux de culture ou d'extraction, avantageusement à partir de cellules de mammifères transfectées par un vecteur portant un ADN complémentaire codant ledit peptide de séquence SEQ is Nº 2, et une étape de purification dudit peptide.

Avantageusement , ledit vecteur porte la séquence d'ADN SEQ ID NO:5

Le peptide tel que précédemment défini, peut être utilisé pour la fabrication d'un médicament à action mitogène, neurotrophique ou stimulant l'activité tyrosine hydroxylase.

Un tel peptide peut être ainsi utilisé pour la fabrication d'un médicament pour la cicatrisation de la peau, la régénération osseuse ainsi que le maintien de l'homéostasie de ces tissus, en particulier pour lutter contre des déséquilibres, tels que l'ostéoporose, ainsi que pour favoriser la vascularisation des structures cérébrales ou du système nerveux .

La présente invention a également pour objet des compositions pharmaceutiques contenant le peptide précédemment défini en association avec un ou plusieurs diluants ou véhicules compatibles et pharmaceutiquement acceptables . De telles compositions sont avantageusement formulées en vue de l'administration entérale ou parentérale par les méthodes usuelles de la galénique.

L'invention sera illustrée, sans être aucunement limitée, par la description qui suit , en référence aux dessins annexés sur lesquels:
La figure 1 est un schéma du plasmide appelé pJK12 utilisé pour transfecter des cellules de mammifères.
La figure 2 représente un gel SDS-PAGE de surnageants respectivement d'un clone cellulaire témoin ( colonne 1) , d'un clone transfecté par le plasmide pJK12 ( colonne 2 ) et de la protéine HARP extraite de cerveaux de bovins.
La figure 3 illustre des effets sur la croissance de neurites de la lignée PC12 respectivement d'un milieu conditionné par des cellules témoins (3a) et d'un milieu conditionné par des cellules transfectées par le plasmide pJK12 (3b).
La figure 4 représente l'activité mitogène de fractions obtenues par chromatographie sur héparine-Sépharose du milieu conditionné par les cellules transfectées par le plasmide pJK12. L'activité mitogène est évaluée par la thymidine tritiée incorporée ( en ordonnée ) tandis que l'absorbance des fractions est mesurée à 280 nm (A 280) . NRHS correspond à la fraction non retenue sur le gel de chromatographie.
La figure 5 illustre l'évolution de l'activité mitogène des fractions obtenues par chromatographie sur Mono S, de la fraction biologiquement active obtenue préalablement sur chromatographie d'héparine-Sépharose. NaCl représente l'évolution de la concentration en NaCl dans le gradient d'élution .
La figure 6 représente la prolifération mesurée par la synthèse d'ADN ( en ordonnée ) en fonction de la concentration en protéine recombinante en ng/ml (en abscisse ).
La figure 7 est une analyse électrophorétique des produits synthétisés par les bactéries transformées par le plasmide codant pour la forme d'HARP longue AEA et courte GKK. Les lysats bactériens produisant les formes AEA (piste 1) ou GKK (piste 3) induites ou non induites à l'IPTG ( piste 2, 3) sont analysés.
La figure 8 est une analyse électrophorétique des fractions AEA (piste 1) et GKK ( piste 2) obtenu après purification. Après migration, le gel est coloré à l'argent.
La figure 9 illustre l'incorporation de thymidine tritiée induite par 10 µl ( 10 ng) ou 5 µl (5ng) de chacune des formes moléculaires de la protéine HARP (AEA et GKK) sont incubés en présence ou non de 125 ng d'héparine dans des cellules épithéliales de cristallin de boeuf.

### EXEMPLES:

### EXEMPLE 1 : PRODUCTION DE LA PROTEINE SELON L'INVENTION PAR CLONAGE ET EXPRESSION DANS DES CELLULES DE MAMMIFERES

### I . Matériels et méthodes

### 1. Matériel

Les produits radiomarqués tels que la thymidine tritiée (3^{H} Tdr) sont commercialisés par Dositek (France) et la Cytosine triphosphate marquée au phosphore 32 est commercialisée par Amersham France.

Les produits de culture de cellules proviennent de la Société Gibco (France), comme le milieu de Eagle modifié par Dulbecco (DMEM), la Généticine (G418), la Pénicilline, la Streptomycine et la Fungizone.

Les sérums de veau nouveau-né et de foetus bovin proviennent de la Société Eurobio (France).

Les lignées cellulaires utilisées sont la lignée PC12 (dérivée de Phéochomocytome) et la lignée de cellules endothéliales capillaires de cerveau bovin (BBC), ainsi que les cellules NIH 3T3.

Les produits permettant d'effectuer les chromatographies proviennent de la Société Pharmacia (Suède) (S-Sépharose, Mono S, heparin-Sépharose).

Le plasmide pAG60 est dû à la générosité du Dr. A. Garapin de l'Institut Pasteur, Paris et est décrit par Colbère et al. J. Mol. Biol., 1981, 150, 1-14.

### 2. Méthodes

### a - Construction du vecteur d'expression eucaryote codant pour la protéine selon l'invention.

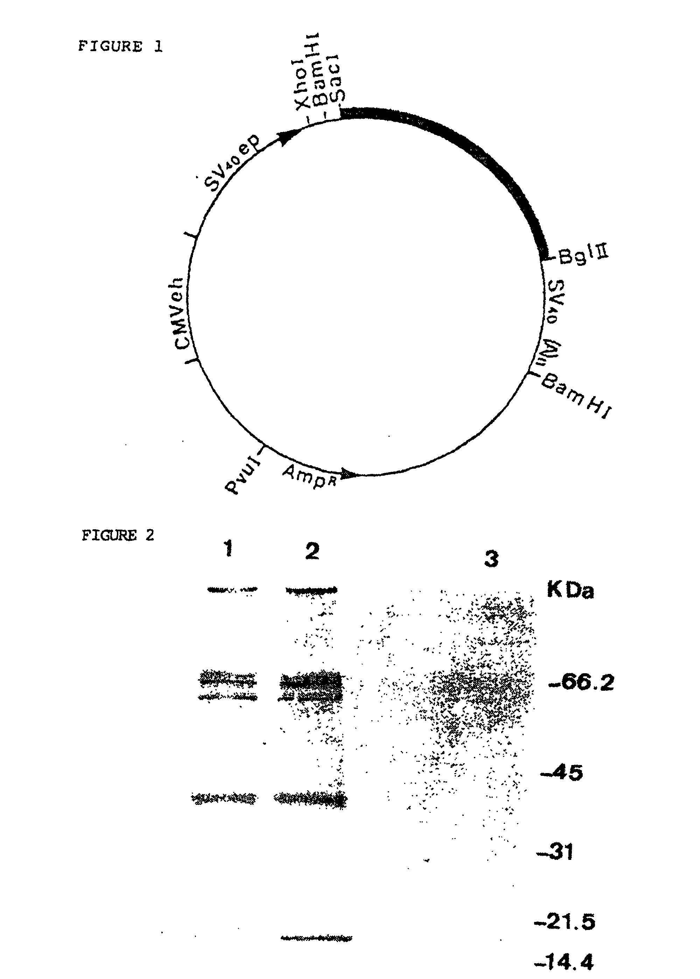

L'isolement du cDNA a été effectué à partir d'une banque de cDNA de cerveau humain établie dans le système lambda gtII et d'une sonde d'oligonucléotides codant pour un morceau de la séquence de HARP (Asp-Cys-Gly-Glu-Trp-Gln). Le gène ainsi isolé de ce clone présente la séquence SEQ ID NO:5. Ce gène a été inséré dans le vecteur d'expression contenant le promoteur SV40 et l'amplificateur du cytomégalovirus humain. Le plasmide ainsi obtenu , de 5,1 Kb , est représenté sur la figure 1. Ce plasmide est appelé pJK12.

### b - Transfections des cellules de mammifères.

Les cellules NIH 3T3 sont cultivées dans du milieu DMEM en présence de 10 % de sérum de veau nouveau-né, de Pénicilline (100 unités par ml) et de Streptomycine (100 µg par ml). Ces cellules sont transfectées en présence de phosphate de calcium comme décrit par Chen et Okayama (Mol. Cell Biol., 1987, 7: 2745-2752).

On réalise les transfections suivantes : 10 µg de pAG60 (plasmide de résistance à la néomycine) ou le mélange contenant 10 µg pJK12 et 1 µg pAG60 dans les cellules NIH 3T3. Après 48 heures, les cellules sont sous-cultivées après dilutions de 1/5e dans un milieu de sélection en présence de l'antibiotique G418 à 400 µg/ml. Ce milieu a été renouvelé tous les 3 jours et après 15 jours, on détecte des colonies de cellules résistant au G418 poussant dans le milieu de culture.

Chaque clone sélectionné est cultivé indépendamment et est étudié. Après 2 sous-cultures, les cellules sont cultivées dans le milieu normal de culture (sans l'antibiotique G418).

### c- Sélection de clone cellulaire sécrétant la protéine selon l'invention après transfection.

Afin de sélectionner un clone exprimant la protéine recombinante, les expériences suivantes sont effectuées :

On récolte le milieu de culture obtenu après 72 heures de culture des cellules du clone transfecté étudié. Ce milieu de culture est complété par du tampon Tris HCl 10 mM, pH 7,5 et est incubé pendant 2 heures à 4° C avec 150 ml d'une solution d'héparine-Sépharose (10 % poids/volume dans un tampon TE 10 mM Tris HCl, pH 7,5, 1mM EDTA). Le gel d'héparine Sépharose a ensuite été lavé 4 fois avec un tampon de 0,6 M NaCl et 2 fois avec le tampon seul.

Les protéines retenues sur l'héparine-Sépharose sont éluées avec 40 µl du tampon d'échantillon d'électrophorèse décrit par Laemmli ( Nature , 1970, 227: 680-685).

L'analyse des protéines sur gel d'électrophorèse en présence de sodium dodécyl sulfate est également effectuée selon le même protocole de Laemmli. Après électrophorèse, les gels sont colorés à l'aide d'argent selon le protocole décrit par Wray et coll,( Anal. Biochem., 1981, 118: 197-203).

### d - Purification de la protéine recombinante selon l'invention.

La purification de la protéine humaine recombinante a été effectuée comme décrit précédemment (Courty et coll, Biochem. Biophys. Res. Commun., 1991, 151: 1312-1318).

En résumé, à partir d'une récolte de 1,2 litres de milieu conditionné , obtenu après plus de soixante douze heures de culture des cellules du clone étudié exprimant la protéine, on ajuste le pH à 6,00 avec 100 mM de phosphate de sodium (tampon A). On centrifuge à 10 000 g pendant 15 minutes pour éliminer les débris cellulaires et les cellules qui flottent dans le milieu et le surnageant est déposé sur une colonne "fast flow" S Sépharose.

Cette résine est lavée avec le tampon A contenant 0,15 M NaCl et les protéines éluées à 0,6 M NaCl dans le même tampon. Cet éluat est ensuite déposé sur une colonne de 1 ml d'héparine-Sépharose à l'aide d'une pompe dont le débit est de 0,5 ml par minute. La colonne d'héparine-Sépharose est préalablement équilibrée en tampon Tris-Hcl 10 mM, pH 7,5 et 0,5 M NaCl. Le matériel non absorbé sur la colonne d'héparine-Sépharose est éliminé ; la colonne est lavée dans le tampon d'équilibration d'une manière exhaustive jusqu'à l'obtention d'une ligne de base d'absorption à 280 nm.

L'élution des protéines absorbées sur la colonne d'héparine-Sépharose est alors réalisée par une augmentation de la concentration en NaCl dans le tampon de lavage. Cette augmentation est faite par paliers successifs jusqu'à la concentration de 2 M NaCl.

Chaque fraction ainsi obtenue est analysée pour son activité mitogène selon le protocole décrit ci-dessous. Les fractions voisines ayant une activité mitogène sont regroupées, dialysées contre le tampon 100 mM phosphate de sodium, pH 6,00 et contenant 0,15 M NaCl. Cette fraction est ensuite déposée sur une colonne de chromatographie Mono S à température ambiante. La séparation des fractions est obtenue par un gradient linéaire de NaCl entre 0,15 et 1 M dans le même tampon. Chaque fraction de 1 ml est étudiée pour son activité mitogène sur des cellules BBC.

### e - Mesures des activités mitogène et neurotrophique.

La mesure des activités mitogènes présentes dans les différentes fractions de chromatographie est effectuée par mesure de l'augmentation de l'incorporation de thymidine tritiée sur des cellules BBC en culture selon le protocole décrit par Courty et coll,( Biochem. Biophys. Res. Commun., 1991, 151: 1312-1318).

La mesure de l'activité neurotrophique sur les cellules PC12 est effectuée après ensemencement de ces cellules à dix mille cellules par boîte de culture de 35 mm de diamètre. Ces cellules sont mises en présence de 3 ml de milieu de culture contenant pour moitié le milieu conditionné obtenu après 72 heures de culture des cellules de clone résistant au milieu de sélection contenant l'antibiotique G418. Après 3 jours, la croissance neuritique est estimée par comparaison avec celle des cellules contrôle.

### f - Microséouençage des protéines.

Le microséquençage de la protéine recombinante selon l'invention est réalisé selon les techniques classiques décrites dans de nombreux ouvrages. Environ 75 mmoles (1,3 µg) de la fraction active repérée par Mono S est déssalée par chromatographie HPLC et injectée dans un microséquenceur en phase gazeuse (Applied Biosystem 470 A). Les dérivés phénylthiohydantoine des acides aminés sont identifiés par chromatographie HPLC (modèle 120 A, Applied Biosystem).

### II. Résultats.

### 1. Séquence du cDNA de la protéine selon l'invention.

La séquence du cDNA de séquence SEQ is Nº 5, codant la protêine selon l'invention, présente des caractéristiques communes à celles publiées par Kretschmer et al, (Growth Factors, 1991, 5: 99-144) , mais , du côté de la région 5' non traduite, la séquence complémentaire à celle décrite entre les bases 130 et 136, soit : GGGAGGG et trois bases additionnelles non décrites en position 137, 138 et 139, soit GAG.

### 2. Mise en évidence de la protéine recombinante selon l'invention.

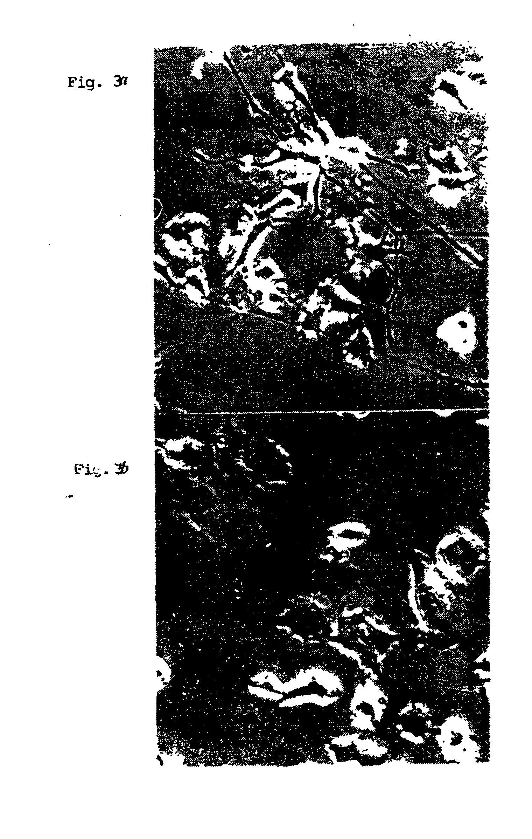

Le milieu, conditionné pendant 72 heures, de deux clones résistant au G418 a été analysé par électrophorèse en SDS. La figure 2 montre l'existence d'un polypeptide de 18 kD présent spécifiquement dans le milieu de culture des cellules transfectées par pJK12 (colonne n° 2) alors que le milieu conditionné par les cellules témoins (transfectées par le vecteur dont est issu pJK12 mais ne portant pas l'ADNc, colonne n° 1 ) ne contient pas cette protéine.

La colonne n°3 correspond à la migration de protéine HARP isolée de cerveau de bovin.

### 3. Mise en évidence de l'activité neurotrophique selon l'invention.

L'activité neurotrophique sur les cellules PC 12 n'est détectée que dans le milieu conditionné par les cellules transfectées par la protéine selon l'invention comme l'illustre la figure 3. On remarque ainsi une croissance de neurites importante dans la figure 3b par rapport à la figure 3a.

### 4. Purification de la protéine selon l'invention et caractérisation de son activité mitogène.

La purification de la protéine à partir du milieu conditionné des cellules transfectées par le plasmide pJK12 est effectuée selon le protocole décrit ci-dessus.

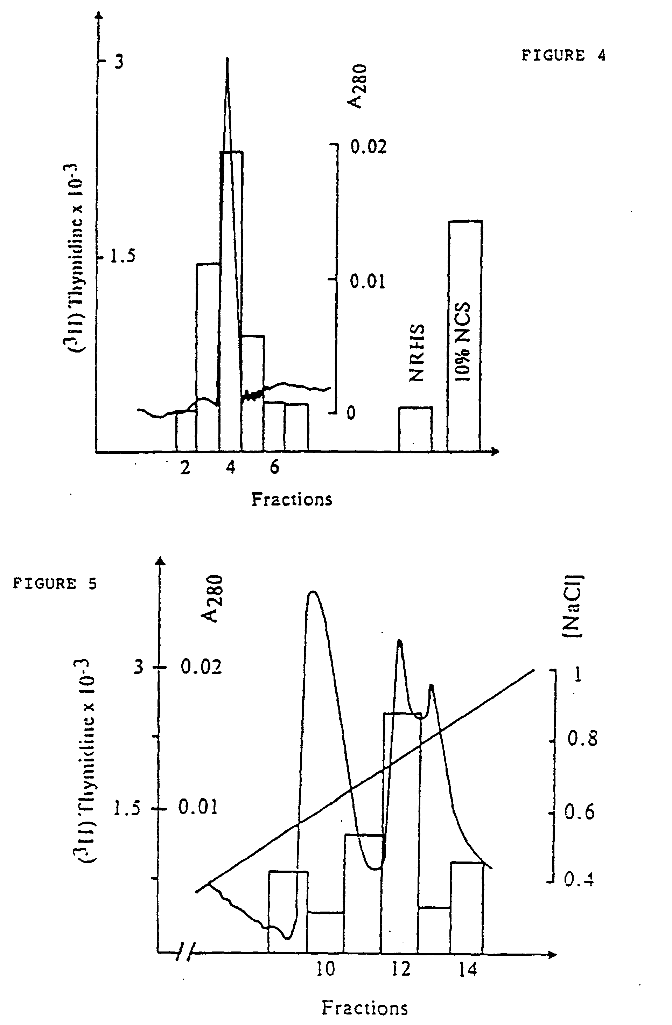

Le profil de l'activité biologique de la protéine obtenue après élution de la chromatographie d'affinité sur héparine-sépharose est présenté dans la figure 4. La fraction éluée à 2 M NaCl stimule l'incorporation de thymidine tritiée dans les cellules BBC.

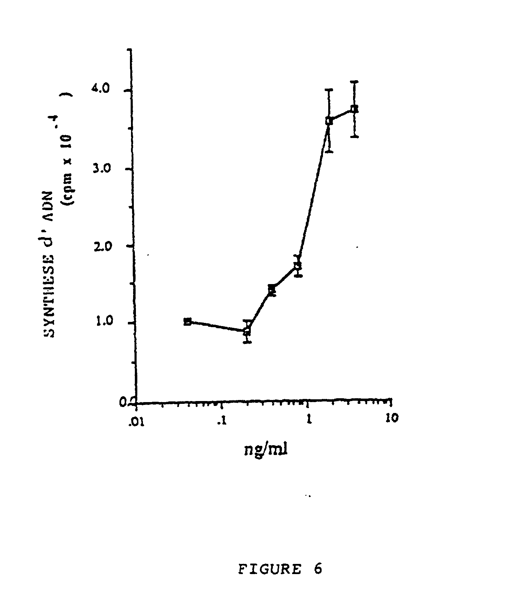

La chromatographie Mono S permet de mieux fractionner cette activité mitogène comme l'illustre la figure 5.

A la concentration de 0,8 M NaCl, une activité mitogène est obtenue et l'étude de cette activité en fonction de la dose est illustrée dans la figure 6 . La dose de protéine nécessaire à ajouter aux cellules BBC en culture dans les conditions opératoires décrites dans la publication de Courty et coll,( Biochem. Biophys. Res. Commun., 1991, 151: 1312-1318) est de 55 pM (1 ng/ml) pour obtenir un effet moitié de l'effet maximal d'incorporation de thymidine tritiée et est de 220 pM (4 ng/ml) pour obtenir le maximum (figure 6).

### 5. Etude de la structure N-terminale de la protéine recombinante selon l'invention.

La fraction biologiquement active éluée à 0,8 M NaCl de la chromatographie Mono S, présente uniquement dans le milieu conditionné des cellules transfectées par le plasmide pJK12 , a été injectée après dessalage par chromatographie HPLC C4 dans un microséquenceur.

La séquence obtenue est la suivante : Aucune séquence GLY-LYS-LYS-GLU-LYS-PRO-GLU-LYS-LYS n'a été détectée.

### EXEMPLE 2

### PRODUCTION DES FORMES LONGUES (AEA) ET COURTES (GKK) DE LA PROTEINE HARP PAR CLONAGE ET EXPRESSION CHEZ E.COLI.

Dans le but de confirmer les relations existant entre la présence de trois acides aminés AEA coté N-terminal et l'activité biologique de la protéine HARP, les deux formes moléculaires de HARP ont été produites chez E. Coli.

### 1) Matériels et Méthodes.

### I. Construction des vecteurs d'expression

Afin d'obtenir les plasmides nécessaires à l'expression de la protéine HARP, deux amplifications géniques in vitro (PCR) ont été réalisées à partir du cDNA humain codant pour la protéine HARP. Ces amplifications ont été réalisées d'une part avec les deux amorces suivantes : générant ainsi un ADN codant pour la forme ayant trois acides aminés de plus côté N-terminal (AEA) et d'autre part avec les deux amorces: générant un ADN codant pour la forme courte (GKK).

Les produits de chaque amplification ont été analysés sur gel d'agarose, coupés avec les enzymes de restriction adéquates puis purifiés selon la technique d'agarase. Le vecteur d'expression procaryote choisi est pMAL-c (Biolabs) qui comportant une protéine de fusion MBP ( maltose binding protein) codé par le gène MAL E. La construction a été réalisée suivant les recommandations décrites par Biolabs.

Le clonage des deux produits d'amplification dans ce vecteur a été réalisé au niveau du polylinker reliant ainsi lors de l'expression la partie C-terminale de la protéine de fusion MBP à la partie N-terminale de la protéine HARP correspondant au site de digestion du facteur Xa ce qui permet ainsi d'obtenir après expression, la protéine HARP.

Les deux vecteurs ainsi obtenus, sont introduits dans la souche Echerichia Coli TB1, selon la technique classique (Maniatis et al; 1982, Laboratory Manual, Cold Spring Harbor Laboratory).

### 2. Criblage des clones obtenus:

Le criblage est réalisé au niveau de l'ADN plasmidique bactérien par recherche du cDNA codant pour l'HARP, puis par la capacité du clone correspondant à produire la protéine recombinante. Ces deux techniques de criblage nous ont permis de choisir deux clones: un codant pour la forme longue de l'HARP (AEA) et un autre codant pour la forme courte de l'HARP (GKK).

### 3. Production de la protéine recombinante:

20 ml provenant d'une pré-culture de chaque clône bactérien produisant les formes longues ou courtes de la protéine HARP, sont incubés dans un litre de milieu 2YT à 37°C, puis incubés en présence de 0,3 mM d'IPTG pendant 3 heures.

Après cette induction, les bactéries sont centrifugées à 4000 g pendant 20 minutes et le culot bactérien résultant est resuspendu dans 50 ml de tampon de lyse: 10 mM phosphate, pH 7, 30mM NaCl, 0,25% Tween 20, 10mM EDTA et 10mM EGTA. Le lysat est ensuite congelé à -20°C pendant une nuit.

Après décongélation, ce lysat est alors soniqué puis clarifié par centrifugation après avoir ajusté la concentration en NaCl à 0,5M final. Le culot ainsi obtenu est resuspendu dans 50 ml d'un tampon constitué de 50mM Tris-HCl, pH 7,5, 6M Guanidine HCl, puis centrifugé 15 minutes à 15000g. Le surnageant est alors dialysé 24 heures à 4°C contre un tampon 20 mM Tris-HCl, pH 7,5, 2mM Mgcl₂ et 1 mM CaCl₂.

Après dialyse le précipité formé est éliminé par centrifugation à 3000 g pendant 5 minutes et le surnageant ajusté à une concentration finale de 0,5 M NaCl, est incubé sous agitation avec 0,5 ml d'héparine-Sépharose une nuit à 4°C. Le gel est ensuite lavé avec un tampon 20 mM hepes, 0,5 M NaCl, puis équilibré avec le tampon 20 mM Tris-HCl, pH 8, 100 mM NaCl et 2 mM CaCl₂. Il est ensuite incubé 24 heures à 4°C en présence du facteur Xa.

Après digestion, les protéines HARP éluées du gel par un tampon 20 mM hepes, pH 7,4 contenant 2 M NaCl.

### 4.Test d'activité mitogène.

L'activité mitogène des fractions obtenues est réalisée par mesure de l'incorporation de thymidine tritiée dans le noyau des cellules épithéliales de cristallin de boeuf (BEL).

1 x 10⁴ cellules BEL et 250 µl de milieu DMEM contenant 10% de sérum de veau foetal, sont ensemensées par puits dans une plaque de culture COSTAR 48 puits.

Après 48 heures de sous culture, le milieu est changé contre du milieu DMEM sans sérum.

Après 48 heures d'incubation, les fractions protéiques à tester sont ajoutées puis après 16 heures de culture dans ces conditions, 1 µCi de thymidine tritiée est ajouté et les cellules sont réincubées pendant 6 heures. La radioactivité TCA précipitable est alors mesurée à l'aide d'un compteur à scintillation.

### II. Résultats.

### 1. Production de la protéine de fusion.

Dans le but de vérifier que les bactéries ont bien intégré les cDNAs codant pour les formes longues et courtes de la molécule HARP, une analyse par électrophorèse des protéines recombinantes produites est réalisée.

0,5 ml d'une culture bactérienne codant pour chacune des formes de l'HARP, induite ou non induite par 0,3 mM IPTG est réalisée suivant le protocole décrit dans Matériels et Méthodes.

Chaque échantillon est centrifugé (4000 g, 20 min) et le culot résultant est resuspendu dans du tampon SDS-PAGE puis analysé par électrophorèse contenant 10% d'acrylamide.

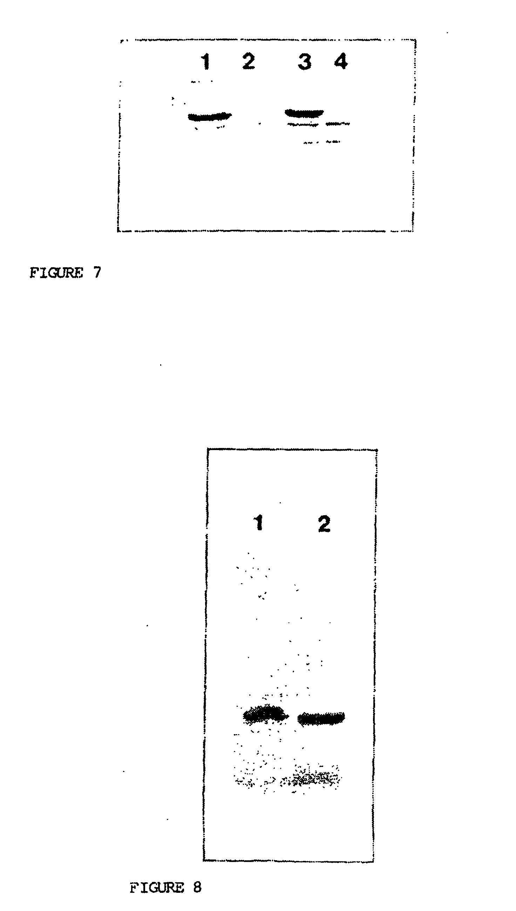

La figure 7 montre le résultat de l'électrophorèse colorée au bleu de coomassie.

L'analyse de ce gel, indique la présence d'une bande majoritaire correspondant à un poids moléculaire de 60 kDa (pistes 1 et 3 ) dans les échantillons ayant été induits par l'IPTG.

Cette protéine de 60 kDa correspond au poids moléculaire de la protéine de fusion constituée de la maltose binding protéine (42 kDa) et d'HARP ( 18 kDa). Aucune bande majoritaire n'est détectée dans les échantillons non induits par l'IPTG ( pistes 2 et 4).

Ce résultat montre que les bactéries ont bien intégrées les plasmides codant pour les formes longues et courtes de la molécule HARP.

### 2.Analyse électrophorétique des formes lonques (AEA) et courtes (GKK) de l'HARP.

100 ng de chacune des protéines obtenues après digestion par le facteur Xa selon la technique décrite dans matériels et méthodes, sont analysées sur gel de polyacrylamide 15 %. Après migration, le gel est révélé par une technique de coloration à l'argent.

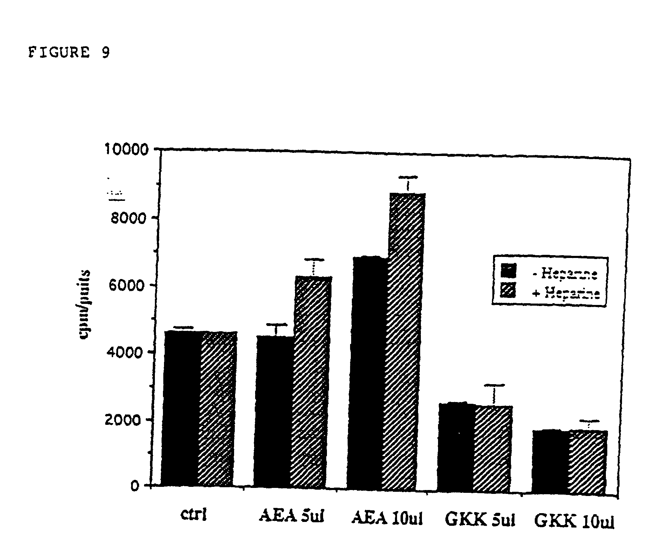

La figure 8 montre la présence d'une bande protéique correspondante à un poids moléculaire de 18 kDa pour chacune des formes AEA ( piste 1) et GKK (piste 2).

### 3. Mesure de l'incorporation de thymidine tritiée induite par les formes AEA et GKK de l'HARP.

Le résultat de l'incorporation de thymidine induite par chacune des formes d'HARP est réprésenté sur la figure 9. La forme longue comportant la séquence AEA à la partie N-terminale induit d'une façon dose dépendante l'incorporation de thymidine dans des cellules BEL. Suivant les concentrations utilisées, cette incorporation semble dépendre de la présence d'héparine (500 ng/ml) dans le milieu de culture. Aucune activité mitogène n'est détectée concernant la forme courte de la molécule HARP (GKK) utilisée à une concentration équivalente (10 ng/ml) à la forme longue (AEA).

### Conclusion

L'activité biologique de la protéine recombinante selon l'invention obtenue par secrétion de cellules de mammifères transfectées par un vecteur portant un ADNc humain ou par production dans E. Coli est attribuée à un polypeptide dont la séquence peptidique présente la caractéristique originale de comporter trois acides aminés supplémentaires du côté N-terminal qui sont ALA-GLU-ALA.

La présence de ces trois acides aminés supplémentaires permet d'obtenir une forme biologiquement active par rapport à la protéine HARP telle que décrite précédemment .

Les activités sont obtenues à des doses très faibles (environ 1 ng/ml soit 55 pM) de protéine , par comparaison aux doses mentionnées dans l'état de la technique.

## Revendications

1. Peptide facteur de croissance ayant une masse de 18 kD,
**caractérisé en ce que** sa séquence est la séquence SEQ ID NO:2

2. Procédé d'obtention du peptide selon la révéndication 1 , par la technique générale d'ingénierie génétique, selon lequel des vecteurs portant un ADN complémentaire codant ledit peptide facteur de croissance sont exprimés et le peptide est ensuite extrait.

3. Procédé selon la revendication 2 comprenant les étapes de sécrètion dudit peptide dans les milieux de culture , à partir de cellules de mammifères transfectées par un vecteur portant un ADN complémentaire codant ledit peptide facteur de croissance, et de purification dudit peptide .

4. Procédé selon la revendication 2, **caractérisé en ce que** lesdits vecteurs sont exprimés dans une bactérie, préférentiellement Escherichia coli.

5. Procédé selon l'une des revendications 2 et 3 **caractérisé en ce que** l'ADN complémentaire possède la séquence SEQ ID NO:5.

6. Utilisation du peptide selon la revendication 1 pour la fabrication d'un médicament pour la cicatrisation de la peau , la régénération osseuse ainsi que le maintien de l'homéostasie de ces tissus , en particulier pour lutter contre des déséquilibres tels que l'ostéoporose, ou à action angiogénique, en particulier pour favoriser la vascularisation des structures cérébrales ou du système nerveux.

7. Composition pharmaceutique contenant une quantité efficace du peptide selon la revendication 1, en association avec un ou plusieurs diluants ou véhicules compatibles et pharmaceutiquement acceptables .

8. Composition selon la revendication 7, **caractérisée en ce qu'**elle est destinée à être utilisée pour l'angiogénèse, en particulier pour favoriser la vascularisation des structurés cérébrales ou du système nerveux.

9. Composition selon la revendication 7, **caractérisée en ce qu'**elle est destinée à être utilisée pour induire la croissance des cellules nerveuses ou pour stimuler l'activité tyrosine hydroxylase.

10. Composition selon la revendication 7, **caractérisée en ce qu'**elle est destinée à être utilisée pour induire le développement de cellules.

11. Composition selon la revendication 7, **caractérisée en ce qu'**elle est destinée à être utilisée pour favoriser la cicatrisation de la peau ou pour maintenir l'homéostatie de ce tissu.

12. Composition selon la revendication 7, **caractérisée en ce qu'**elle est destinée à être utilisée pour favoriser la régénération osseuse ainsi que le maintien de l'homéostatie de ce tissu, en particulier pour lutter contre des déséquilibres, tels que l'ostéoporose.

13. Composition selon l'une des revendications 7 à 12 présentée en vue de l'administration entérale ou parentérale.

## Patentansprüche

1. Wachstumsfaktorpeptid mit einer Masse von 18 kD, **dadurch gekennzeichnet, dass** seine Sequenz die Sequenz SEQ ID NO: 2 ist.

2. Verfahren zur Gewinnung des Peptids gemäß Anspruch 1 durch die allgemeine gentechnische Methode, gemäß der Vektoren, die eine komplementäre DNA tragen, die das Wachstumsfaktorpeptid codiert, exprimiert werden und das Peptid anschließend extrahiert wird.

3. Verfahren gemäß Anspruch 2 mit den Schritten der Sekretion des Peptids in die Kulturmedien ausgehend von Säugerzellen, die mit einem Vektor transfiziert sind, der eine komplementäre DNA trägt, die das Wachstumsfaktorpeptid codiert, und der Reinigung des Peptids.

4. Verfahren gemäß Anspruch 2, **dadurch gekennzeichnet, dass** die Vektoren in einem Bakterium, vorzugsweise *Escherichia coli*, exprimiert werden.

5. Verfahren gemäß einem der Ansprüche 2 und 3, **dadurch gekennzeichnet, dass** die komplementäre DNA die Sequenz SEQ ID NO: 5 besitzt.

6. Verwendung des Peptids gemäß Anspruch 1 zur Herstellung eines Medikaments zur Vernarbung der Haut, zur Knochenregeneration sowie zur Aufrechterhaltung der Homöostase dieser Gewebe, insbesondere zur Bekämpfung von Ungleichgewichten, wie Osteoporose, oder mit angiogener Wirkung, insbesondere zur Begünstigung der Gefäßversorgung der Hirnstrukturen oder des Nervensystems.

7. Pharmazeutische Zusammensetzung, die eine wirksame Menge des Peptids gemäß Anspruch 1 in Verbindung mit einem oder mehreren verträglichen und pharmazeutisch annehmbaren Verdünnungsmitteln oder Trägern enthält.

8. Zusammensetzung gemäß Anspruch 7, **dadurch gekennzeichnet, dass** sie zur Verwendung für die Angiogenese, insbesondere zur Begünstigung der Gefäßversorgung der Hirnstrukturen oder des Nervensystems, bestimmt ist.

9. Zusammensetzung gemäß Anspruch 7, **dadurch gekennzeichnet, dass** sie zur Verwendung für die Induktion des Wachstums von Nervenzellen oder für die Stimulation der Tyrosin-Hydroxyiase-Aktivität bestimmt ist.

10. Zusammensetzung gemäß Anspruch 7, **dadurch gekennzeichnet, dass** sie zur Verwendung für die Induktion der Zellentwicklung bestimmt ist.

11. Zusammensetzung gemäß Anspruch 7, **dadurch gekennzeichnet, dass** sie zur Verwendung für die Begünstigung der Narbenbildung der Haut oder für die Aufrechterhaltung der Homöostase dieses Gewebes bestimmt ist.

12. Zusammensetzung gemäß Anspruch 7, **dadurch gekennzeichnet, dass** sie zur Verwendung für die Begünstigung der Knochenregeneration sowie zur Aufrechterhaltung der Homöostase dieses Gewebes, insbesondere zur Bekämpfung von Ungleichgewichten, wie Osteoporose, bestimmt ist.

13. Zusammensetzung gemäß einem der Ansprüche 7 bis 12, die für die enterale oder parenterale Verabreichung geeignet ist.

## Claims

1. Growth factor peptide **characterized in that** its sequence is SEQ ID N° : 2.

2. Method of preparation of a peptide according to claim 1 by the general technique of genetic engineering, according to which vectors carrying a complementary DNA encoding said acting peptide are expressed and the peptide is then extracted.

3. Method according to claim 2 comprising the steps of secretion of said peptide in culture media from mammalian cells transfected by a vector carrying a complementary DNA encoding said acting peptide, and of purification of said peptide.

4. Method according to claim 2, **characterized in that** said vectors are expressed in a bacteria, preferably Escherichia coli.

5. Method according to one of claims 2 and 3, **characterized in that** the complementary DNA has the sequence SEQ ID N° : 5.

6. Use of a peptide according to claim 1 for producing a drug for skin healing bone regeneration or regulation of the homeostasis of these tissues, in particular to control imbalances such as osteoporosis, or with an angiogenic action, in particular to enhance the vascularization of brain structures or of the nervous system.

7. Pharmaceutical composition containing an effective quantity of a peptide according to claim 1 in combination with one or more compatible and pharmaceutically acceptable diluents or carriers.

8. Composition according to claim 7, **characterized in that** it is intented to be used for angiogenesis, in particular to enhance the vascularization of brain structures or of the nervous system.

9. Composition according to claim 7, **characterized in that** it is intended to be used to induce growth of nerve cells or to stimulate tyrosine hydroxylase activity.

10. Composition according to claim 7, **characterized in that** it is intended to be used to induce cell development.

11. Composition according to claim 7, **characterized in that** it is intended to be used to improve skin healing or to regulate the homeostasis of this tissue.

12. Composition according to claim 7, **characterized in that** it is intended to be used to improve bone regeneration and to regulate the homeostasis of this tissue, in particular to control imbalances such as osteoporosis.

13. Composition according to one of claims 7 to 12 formulated for enteral or parenteral administration.


### LISTE DE SEQUENCES

(1) INFORMATION GENERALE:
   (1) DEPOSANT:
      (A) NOM: Universite Paris Val de Marne
      (B) RUE: avenue du general de gaulle
      (C) VILLE: Creteil
      (E) PAYS: France
      (F) CODE POSTAL: 94010
   (ii) TITRE DE L' INVENTION: Facteurs de croissance de la famille de l'HARP ,procedes d'obtention et applications
   (iii) NOMBRE DE SEQUENCES: 8
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 12 acides aminés
      (B) TYPE: acide aminé
      (D) CONFIGURATION: linéaire
   (ii) TYPE DE MOLECULE: peptide
   (iii) HYPOTHETIQUE: NON
   (xi) DESCRIPTION DE LA SEQUENCE: SEQ ID NO: 1:
   Ala Glu Ala Gly Lys Lys Glu Lys Pro Glu Lys Lys
   1               5                   10
(2) INFORMATION POUR LA SEQ ID NO: 2:
   (i) CARACTERISTIQUES DE LA SEQUENCE:
      (A) LONGUEUR: 139 acides aminés
      (B) TYPE: acide aminé
      (D) CONFIGURATION: linéaire
   (ii) TYPE DE MOLECULE: peptide
   (iii) HYPOTHETIQUE: NON
   (xi) DESCRIPTION DE LA SEQUENCE: SEQ ID NO: 2:
   Ala Glu Ala Gly Lys Lys Glu Lys Pro Glu Lys Lys Val Lys
   1               5                   10
   Lys Ser Asp Cys Gly Glu Trp Gln Trp Ser Val Cys Val Pro
   15                  20                  25
   Thr Ser Gly Asp Cys Gly Leu Gly Thr Arg Glu Gly Thr Arg
       30                  35                  40
   Thr Gly Ala Glu Cys Lys Gln Thr Met Lys Thr Gln Arg Cys
           45                  50                  55
   Lys Ile Pro Cys Asn Trp Lys Lys Gln Phe Gly Ala Glu Cys
               60                  65                  70
   Lys Tyr Gln Phe Gln Ala Trp Gly Glu Cys Asp Leu Asn Thr
                   75                  80
   Ala Leu Lys Thr Arg Thr Gly Ser Leu Lys Arg Ala Leu His
   85                  90                  95
   Asn Ala Glu Cys Gln Lys Thr Val Thr Ile Ser Lys Pro Cys
       100                 105                 110
   Gly Lys Leu Thr Lys Pro Lys Pro Gln Ala Glu Ser Lys Lys
           115                 120                 125
   Lys Lys Lys Glu Gly Lys Lys Gln Glu Lys Met Leu Asp
               130                 135
(2) INFORMATION POUR LA SEQ ID NO: 3:
   (i) CARACTERISTIQUES DE LA SEQUENCE:
      (A) LONGUEUR: 16 acides aminés
      (B) TYPE: acide aminé
      (D) CONFIGURATION: linéaire
   (ii) TYPE DE MOLECULE: peptide
   (iii) HYPOTHETIQUE: NON
   (xi) DESCRIPTION DE LA SEQUENCE: SEQ ID NO: 3:
   Ala Val Asp Thr Ala Glu Ala Gly Lys Lys Glu Lys Pro Glu
   1               5                   10
   Lys Lys
   15
(2) INFORMATION POUR LA SEQ ID NO: 4:
   (i) CARACTERISTIQUES DE LA SEQUENCE:
      (A) LONGUEUR: 143 acides aminés
      (B) TYPE: acide aminé
      (D) CONFIGURATION: linéaire
   (ii) TYPE DE MOLECULE: peptide
   (iii) HYPOTHETIQUE: NON
   (xi) DESCRIPTION DE LA SEQUENCE: SEQ ID NO: 4:
   Ala Val Asp Thr Ala Glu Ala Gly Lys Lys Glu Lys Pro Glu
   1               5                   10
   Lys Lys Val Lys Lys Ser Asp Cys Gly Glu Trp Gln Trp Ser
   15                  20                  25
   Val Cys Val Pro Thr Ser Gly Asp Cys Gly Leu Gly Thr Arg
       30                  35                  40
   Glu Gly Thr Arg Thr Gly Ala Glu Cys Lys Gln Thr Met Lys
           45                  50                  55
   Thr Gln Arg Cys Lys Ile Pro Cys Asn Trp Lys Lys Gln Phe
               60                  65                  70
   Gly Ala Glu Cys Lys Tyr Gln Phe Gln Ala Trp Gly Glu Cys
                   75                  80
   Asp Leu Asn Thr Ala Leu Lys Thr Arg Thr Gly Ser Leu Lys
   85                  90                  95
   Arg Ala Leu His Asn Ala Glu Cys Gln Lys Thr Val Thr Ile
       100                 105                 110
   Ser Lys Pro Cys Gly Lys Leu Thr Lys Pro Lys Pro Gln Ala
           115                 120                 125
   Glu Ser Lys Lys Lys Lys Lys Glu Gly Lys Lys Gln Glu Lys
               130                 135                 140
   Met Leu Asp
(2) INFORMATION POUR LA SEQ ID NO: 5:
   (i) CARACTERISTIQUES DE LA SEQUENCE:
      (A) LONGUEUR: 995 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (iii) HYPOTHETIQUE: NON
   (xi) DESCRIPTION DE LA SEQUENCE: SEQ ID NO: 5:
   GGGAGGGAGA GCGCCCAGCC TTCGTCCTCC TGGCCCGCTC CTCTCATCCC TCCCATTCTC    60
   CATTTCCCTT CCGTTCCCTC CCTGTCAGGG CGTAATTGAG TCAAAGGCAG GATCAGGTTC   120
   CCCGCCTTCC AGTCCAAAAA TCCCGCCAAG AGAGCCCCAG AGCAGAGGAA AATCCAAAGT   180
   GGAGAGAGGG GAAGAAAGAG ACCAGTGAGT CATCCGTCCA GAAGGCGGGG AGAGCAGCAG   240
   CGGCCCAAGC AGGAGCTGCA GCGAGCCGGG TACCTGGACT CAGCGGTAGC AACCTCGCCC   300
   CTTGCAACAA AGGCAGACTG AGCGCCAGAG AGGACGTTTC CAACTCAAAA ATGCAGGCTC   360
   AACAGTACCA GCAGCAGCGT CGAAAATTTG CAGCTGCCTT CTTGGCATTC ATTTTCATAC   420
   TGGCAGCTGT GGATACTGCT GAAGCAGGGA AGAAAGAGAA ACCAGAAAAA AAAGTGAAGA   480
   AGTCTGACTG TGGAGAATGG CAGTGGAGTG TGTGTGTGCC CACCAGTGGA GACTGTGGGC   540
   TGGGCACACG GGAGGGCACT CGGACTGGAG CTGAGTGCAA GCAAACCATG AAGACCCAGA   600
   GATGTAAGAT CCCCTGCAAC TGGAAGAAGC AATTTGGCGC GGAGTGCAAA TACCAGTTCC   660
   AGGCCTGGGG AGAATGTGAC CTGAACACAG CCCTGAAGAC CAGAACTGGA AGTCTGAAGC   720
   GAGCCCTGCA CAATGCCGAA TGCCAGAAGA CTGTCACCAT CTCCAAGCCC TGTGGCAAAC   780
   TGACCAAGCC CAAACCTCAA GCAGAATCTA AGAAGAAGAA AAAGGAAGGC AAGAAACAGG   840
   AGAAGATGCT GGATTAAAAG ATGTCACCTG TGGAACATAA AAAGGACATC AGCAAACAGG   900
   ATCAGTTAAC TATTGCATTT ATATGTACCG TAGGCTTTGT ATTCAAAAAT TATCTATAGC   960
   TAAGTACACA ATAAGCAAAA ACAACCAATT TGGGT                              995
(2) INFORMATION POUR LA SEQ ID NO: 6:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 6:
   GCTGAAGCAG GGAAGAAAGA G 21
(2) INFORMATION POUR LA SEQ ID NO: 7:
   (1) CARACTERISTIQUES DE LA SEQUENCE:
      (A) LONGUEUR: 29 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 7:
   GGTCTCGAGT ATGTTCCACA GGTGACATC 29
(2) INFORMATION POUR LA SEQ ID NO: 8:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 8:
   GGGAAGAAAG AGAAACCAGA A 21